# EUROPEAN PATENT SPECIFICATION

(11) **EP 1 755 642 B1**
(45) Date of publication and mention of the grant of the patent: **13.01.2010**
(21) Application number: 05735990.3
(22) Date of filing: 15.04.2005
(51) Int. Cl.: A61K 38/17, A61K 39/395, A61P 7/02

(54) **ANNEXIN V FOR PREVENTING PLAQUE RUPTURE**
ANNEXIN ZUR PRÄVENTION VON DER RUPTUR DES PLAQUES
L'ANNEXINE V POUR LA PREVENTION DE LA RUPTURE DE LA PLAQUE

(30) Priority: 15.04.2004 US 521385 P
(43) Date of publication of application: 28.02.2007
(73) Proprietor: ATHERA BIOTECHNOLOGIES AB, 17177 Stockholm (SE)
(72) Inventor: FROSTEGÅRD, Anna, S-112 42 Stockholm (SE); FROSTEGÅRD, Johan, S-112 42 Stockholm (SE)
(74) Representative: Wright, Andrew John
(86) International application number: PCT/GB2005/001451
(87) International publication number: WO 2005/099744

(56) References cited:
- EP-A- 1 153 611
- WO-A-02/067857
- US-A1- 2003 152 513
- US-A1- 2003 170 241
- MARI C ET AL: "Annexin V, a new therapeutic tool in atherosclerosis" JOURNAL OF NUCLEAR MEDICINE, vol. 43, no. 5 Supplement, May 2002 (2002-05), page 7P, XP009051427 & 49TH ANNUAL MEETING OF THE SOCIETY OF NUCLEAR MEDICINE; LOS ANGELES, CA, USA; JUNE 15-19, 2002 ISSN: 0161-5505
- THIAGARAJAN PERUMAL ET AL: "Inhibition of arterial thrombosis by recombinant annexin V in a rabbit carotid artery injury model" CIRCULATION, [Online] vol. 96, no. 7, 1997, pages 2339-2347, XP002338645 ISSN: 0009-7322 Retrieved from the Internet: URL:http://circ.ahajournals.org/cgi/conten t/abstract/96/7/2339> [retrieved on 2005-07-27]
- SHERER Y ET AL: "Immunomodulation for treatment and prevention of atherosclerosis" AUTOIMMUNITY REVIEWS 2002 NETHERLANDS, vol. 1, no. 1-2, 2002, pages 21-27, XP002338646 ISSN: 1568-9972
- ALVES J D ET AL: "Atherosclerosis, oxidative stress and auto-antibodies in systemic lupus erythematosus and primary antiphospholipid syndrome" IMMUNOBIOLOGY, FISCHER, STUTTGART, DE, vol. 207, no. 1, 2003, pages 23-28, XP004954257 ISSN: 0171-2985
- GOLDENBERG H B ET AL: "Human antibody to phosphorylcholine is bactericidal against Haemophilus influenzae." ABSTRACTS OF THE GENERAL MEETING OF THE AMERICAN SOCIETY FOR MICROBIOLOGY, vol. 103, 2003, pages D-116 URL, XP009051386 & 103RD AMERICAN SOCIETY FOR MICROBIOLOGY GENERAL MEETING; WASHINGTON, DC, USA; MAY 18-22, 2003 ISSN: 1060-2011
- CEDERHOLM ANNA ET AL: "Decreased binding of annexin V to endothelial cells - A potential mechanism in atherothrombosis of patients with systemic lupus erythematosus" ARTERIOSCLEROSIS THROMBOSIS AND VASCULAR BIOLOGY, vol. 25, no. 1, January 2005 (2005-01), pages 198-203, XP009051391 ISSN: 1079-5642

## Description

### FIELD OF THE INVENTION

This invention relates to the fields of atherosclerosis and atherothrombosis. The invention relates specifically to novel mechanisms for prevention or inhibition of plaque rupture.

### DESCRIPTION OF RELATED ART

There is an interrelationship between atherosclerosis and atherothrombosis.

Atherosclerosis has many characteristics of an inflammatory disease, including abundance of inflammatory cells and production of pro-inflammatory cytokines in lesions^{1,2}.

The increased risk of mortality due to cardiovascular diseases, specifically in systemic lupus erythematosus (SLE) patients, is a major clinical problem.

Cardiovascular diseases in SLE patients is associated with both traditional risk factors like dyslipidemia, and non-traditional risk factors including increased oxidation of low density lipoprotein (oxLDL), raised activity in the tumour necrosis factor (TNF)-system (closely associated with dyslipidemia), systemic inflammation as determined by CRP, homocystein and anti-phospholipid antibodies (aPL)³⁻⁷. Anti-phospholipid may cause the anti-phospholipid antibody syndrome (APS), common in SLE patients and characterized by recurrent pregnancy loss and recurrent thrombosis^{8,9}. Different forms of anti-phospholipids have also been implicated in cardiovascular diseases in the general population^{10,11}.

Annexins share the property of binding calcium and negatively charged phospholipids, both of which are required for blood coagulation. Recently, Annexin V has been implicated in anti-phospholipid antibody syndrome since some anti-phospholipid antibodies disrupt the Annexin-V antithrombotic shield in the placenta, predisposing to placental micro thrombosis and recurrent miscarriage¹²⁻¹⁴.

The binding of Annexin V to activated platelets and to damaged cells probably explains the selective retention of the protein in thrombi. This has been shown in experimental animal models of venous and arterial thrombosis¹⁵ and labelled Annexin has been proposed for medical imaging of vascular thrombi in humans, with reduced noise and increased safety¹⁶.

It is reported¹⁸ that exogenously administered Annexin V has affinity for stressed or apoptotic cells and that this property can be used to create a multifunctional molecular probe useful in imaging and in the treatment of unstable plaques, in which the Annexin is coupled to targeting and effector molecules. Effector molecules are used to selectively kill or inhibit stressed or apoptotic cells. However, there is no indication that Annexin V alone (i.e. without targeting or effector molecules) would have any effect on unstable plaques.

Annexin V has been suggested to decrease apoptotic activity of cells in atherosclerotic plaques of apoE -/- mouse models¹⁹. However, the report fails to show any effect on plaque rupture, and says nothing about the human model.

Annexin V has also been shown to inhibit arterial thrombosis in rabbits²⁰ and that this effect is mediated via its interaction with phosphatidylserine (PS) on platelets. There is no discussion in this reference of atherothrombosis or plaque rupture, nor any data relating to humans.

A major problem associated with the therapeutic use of Annexin V in coagulation disorders is its short half-life in the circulation, estimated in experimental animals to be 5 to 15 minutes¹⁵,¹⁷; Annexin V has a short half-life in the circulation of humans.

In EP1379266 "Modified Annexin proteins and methods for preventing thrombosis" is claimed a polyethylene glycol-modified Annexin protein to be used to prevent thrombosis without increasing haemorrhage. The half-life of Annexin V is improved by using a PEG conjugate in a method to prevent binding of the prothrombinase complex necessary for thrombus formation.

In the present invention we have shown that Annexin V may stabilize atherosclerotic plaque. When Annexin V or an N-terminal fragment of Annexin V is administered according to the invention, preferably by injection, it will bind to the endothelial plaque on a first passage. The short half-life of Annexin V in the circulation is thus not a problem as is the case in EP1379266. A composition for injection comprising Annexin V or an N-terminal fragment of Annexin V with or without additives will thus prevent atherothrombosis by stabilizing the carotid plaque through an instant binding.

Immunoglobulin G or IgG is a protein found in adults in normal concentrations ranging from 900 mg/dl to 2400 mg/dl. This is approximately 20% of the total protein found in serum or plasma. IgG has a half life of 23 days. It contributes immunity to bacteria, viruses, parasites, some fungi and provides antibody activity in tissue. IgG is able to activate complement which triggers the inflammatory response and is a subject unto itself. IgG provides protection to various viruses and such for the animal through inoculations or natural barnyard exposure. Once exposure occurs a memory is developed which allows the body to rapidly manufacture needed antibodies to fight specific infections. In man IgG is transferred to the baby through the placenta. If IgG is not present or in low levels, mammals have poor defences against any infectious agent to which it might be exposed.

Intravenous immunoglobulin preparations (e.g. IGIV; Baxter and others is a highly purified preparation of IgG commercially available and is used in the treatment of patients who have no, or very low levels of antibody production. Immunoglobulin preparations include those available from the following manufacturers: Baxter (US) eg Gammagard®, Isiven (Antimo Naples, Italy), Omrix (Tel-Hashomer, Israel), Miles (Biological Products Division, West Heaven, CT), Sclavo (Lucca, Italy), Sandoz (Novartis, Basel, Switzerland eg Sandoglobulin®), Biotest Diagnostic Corporation (Deville, NJ). Examples of immunoglobulin preparations are Gammagard S/D®, Gammar IV®, Gammar-P IV® Gammimune N®, Iveegam®, Panglobulin®, Polygam S/D®, Sandoglobulin®, Venoglobulin®. Immunoglobulin preparations typically contain some IgM as well as IgG. Trace amounts of IgM are present in Gammagard®. Pentaglobin (Biotest) is an enriched IgM preparation which has been used for treatment of SARS.

Such IvIg preparations from pooled plasma derived from many donors is often used also in autoimmune conditions, where one recognized mode of action is the presence of anti-idiotypic antibodies, e g antibodies reacting with and neutralizing other antibodies that are pathogenic.

In US6613328 is described a method for treating thrombosis diseases with von Willebrand factor specific antibodies. Humanized antibodies are specifically produced. There is however no information that readily available immunoglobulins such as IGIV or subfractions of these immunoglobulins can be used to prevent atherothrombosis or plaque rupture. Furthermore, there is no information in the literature that IgG can inhibit the binding of antibodies to native Annexin V, which is a possible reason for the decreased Annexin V-binding to endothelium, presented in this invention.

### SUMMARY OF THE INVENTION

The invention provides the use of Annexin V, optionally in the form of a salt, to prevent plaque rupture in humans.

The invention also provides the use of an intravenous immunoglobulin (IVIG) preparation to prevent plaque rupture in a human SLE patient.

The invention also provides the use of a purified anti Ig antibody subfraction of a pooled immunoglobulin preparation containing anti-aPAF antibodies and/or anti-IgG antibodies with the capacity to decrease the inhibitory effect of plasma of a systemic lupus erythematosus (SLE) patient on binding of Annexin V to endothelial cells, to prevent plaque rupture in a human SLE patient.

Thus, disclosed herein are uses for preventing plaque rupture by administration of a compound that restores the binding of Annexin V to plaque, which may be by inhibiting Ig responsible for inhibiting the binding. This treatment is accomplished either by giving a dose of Annexin V preferably by IV injection; or by IV administration of immunoglobulins or a subfraction of immunoglobulins that act to promote the binding of Annexin V to plaque, possibly by inhibiting the Annexin V binding to antibodies. Immunoglobulin (IGIV; Baxter) or other commercially available preparations (examples of which are rioted above) as well as affinity purified subfractions, which are known by the art for treatment or prevention of infections and severe autoimmune conditions, can be used. An affinity purified subfraction of immunoglobulins is preferable.

The active component (Annexin V or immunoglobulin subtraction) is administered to a subject at risk of atherothrombosis using a pharmaceutical composition having an effective amount of the active component. The pharmaceutical composition can be administered intravenously or by other routes as a treatment of patients belonging to a risk group. A preferable risk group is systemic lupus erythematosus (SLE) patients. The treatment can be repeated at optimal time intervals.

### DETAILED DESCRIPTION OF THE INVENTION

The risk of plaque rupture is strongly raised when Annexin V binding to endothelium is decreased as a consequence of antibodies inhibiting the Annexin V-plaque binding. Restoring of the Annexin V binding by administration of Annexin V (or fragments) or by administering immunoglobulins, preferably a subfraction of immunoglobulins (ie a subfraction of a pooled immunoglobulin preparation as discussed above), that inhibit other antibodies (for example Annexin-V-binding antibodies) that decrease the plaque-Annexin binding provides novel proposed therapies for plaque rupture, the main cause of cardiovascular disease.

A first aspect of the invention provides the use of the Annexin V protein optionally in the form of a salt, in the manufacture of a pharmaceutical composition to prevent plaque rupture.

The term Annexin V is well known to those skilled in the art and is used, for example, in documents cited above, for example EP 1 379 266. As will be apparent to the skilled person, the N-terminal fragment of Annexin V is large enough to be recognisable by the skilled person as a fragment of Annexin V (rather than, for example, a fragment of another annexin.

The pharmaceutical composition may comprise an effective amount of the Annexin V protein optionally in combination with a carrier and additives. Suitable carriers and additives which can be used will be well known to those skilled in the art, including the examples used in EP 1 379 266. The salt can be a pharmaceutically acceptable acid addition salt where the counter ion is, for example, chloride, acetate.

The effective amount of the Annexin V in the pharmaceutical composition may be determined from a diagnostic status analysis of the Annexin V-endothelium binding. Thus, the dosage may be determined by assessing the state of Annexin V-endothelium binding in the patient. Thus, Annexin V-endothelium binding may be assessed by assessing the effect of the patient's serum on binding of Annexin V to endothelium, for example using a technique such as that used in the Examples to assess the effect of patient plasma on binding of Annexin V to cultured endothelial cells (see the "Culture of endothelial cells binding; binding of Annexin V" and "Results" sections). Immunohistochemical staining of biopsy plaque material from the patient (as described in the "Immunohistochemical staining of human atherosclerotic plaque" section of the Examples) may also be used. Imaging techniques using labelled Annexin (see discussion of reference 16 above (WO 95/343315)) may also be used. As will be understood by the skilled person, should the patient be found to have very low Annexin V binding then a higher dosage of Annexin V may be required than if the patient is found to have Annexin V binding closer to that found in a normal person.

The invention provides a treatment of a subject at risk of plaque rupture, comprising administering to said subject a pharmaceutical composition comprising an effective amount of Annexin V, optionally in the form of a salt.

The subject at risk may be a systemic lupus erythematosus (SLE) patient. The SLE patient may further have risk factors as discussed above, for example dyslipidemia, increased oxidation of low density lipoprotein (oxLDL), raised activity in the tumour necrosis factor (TNF)-system (closely associated with dyslipidemia), systemic inflammation as determined by CRP, homocystein and anti-phospholipid antibodies (aPL). The SLE patient may further show recurrent thrombosis or frequent repeating atherothrombosis, or have survived one or more manifestations of cardiovascular disease, for example thromboembolic, not hemorrhagic or vasculitic stroke, myocardial infarction, angina pectoris or intermittent claudication. The subject at risk may be a patient that has or has had, or is at risk of, a pneumococcal infection; or may be a patient with vulnerable plaques, as identified based on symptoms and clinical evaluation indicative of imminent cardiovascular disease such as unstable angina, other forms of severe angina, or transient ischemic attacks (TIA). An Annexin V-endothelium binding assessment, as discussed above, may be used in assessing risk.

Also described herein is the use of a purified subfraction of immunoglobulins (ie a purified subfraction of a pooled immunoglobulin preparation, as discussed above) with the capacity to inhibit antibodies binding to Annexin V. Such a subfraction may be prepared using techniques indicated above, for example using affinity purification. The purified subfraction may be an anti IgG antibody subfraction, for example a subfraction selected by affinity binding to IgG, in particular to the IgG constant domain.

Also disclosed herein is the use of a purified subfraction of immunoglobulins (ie a purified subfraction of a pooled immunoglobulin preparation, as discussed above) with the capacity to promote binding of Annexin V to endothelium (or endothelial cells, for example in culture). Such a subfraction may be prepared using techniques indicated above, for example using affinity purification. The subfraction may be anti Ig antibody subfraction, for example a subtraction selected by affinity binding to IgG, in particular to the IgG constant domain. Such a subfraction may contain anti-aPAF, anti-aLPC, anti-aPS or anti-a-pneumococcal vaccine which may reduce levels of aPAF, aLPC, aPS or antibodies to pneumococcal vaccine, depletion of which was found to increase Annexin V binding (see the Examples). The capacity of the subfraction to promote binding of Annexin V to endothelium may be assessed using an Annexin V-endothelium binding assay as referred to above and in the Examples. For example, an appropriate subfraction may be one which decreases the inhibitory effect of plasma from SLE cases (high antiphospholipid antibodies (aPLs) titer serum) on binding of Annexin V to endothelial cells. As noted in the examples, we have found a statistical correlation between levels of aPC-BSA and aPC-KLH and Annexin V binding. It is considered that aPC IgG and/or IgM may bind to some IgG (see, for example, Halpern et al (1991) J Clin Invest 88(2), 476-482) and are most likely produced by B1 cells, a B cell subtype that may also neutralize B2 cells that produce IgG.

Also disclosed herein is a purified subtraction of the invention for use for preventing plaque rupture.

A further aspect of the invention provides the use of a purified subfraction according to the preceding aspect of the invention or the use of a commercially available immunoglobulin preparation (ie a pooled immunoglobulin preparation, as discussed above) in the manufacture of a medicament to prevent plaque rupture.

Also disclosed herein is a treatment of a subject at risk of plaque rupture, comprising administering to said subject a pharmaceutical composition comprising an effective amount of immunoglobulins (ie of a pooled immunoglobulin preparation as discussed above) or a purified subtraction of immunoglobulins (examples of which are indicated above).

Preferences for the subject to be treated (or for which the medicament is for treating) are discussed above. The subject may be, for example, a systemic lupus erythematosus (SLE) patient or a patient who has, has had or is at risk of a pneumococcal infection; or a patient with vulnerable plaques and/or unstable angina.

The invention will now be described in more detail by reference to the following, non-limiting, Figures and Examples.

### Figures:

**Figure 1****: Effect of pre-incubation of high antiphospholipid antibodies (aPLs) titer serum with human pooled immunoglobulin Gammagard® on Annexin V binding to human umbilical endothelia cells (HUVECs): flow cytometry analysis after 24 hrs culture.**

| IVIG pre-incubated with serum at | Median fluorescence intensity (MFI) of Annexin V binding |
|---|---|
| 0 mg/ml | 649 |
| 2.5 mg/ml | 913 |
| 5 mg/ml | 1269 |
| 10 mg/ml | 1382 |

**Figure 2****: Effect of pre-incubation of high antiphospholipid antibodies (aPLs) titer serum with pneumococcal capsular polysaccharide on Annexin V binding to HUVECs: flow cytometry analysis after 24 hrs culture.**

| High aPLs titer serum pre-incubated with penumococcal capsular polysaccharide at | Median Fluorescence Intensity (MFI) of Annexin V binding |
|---|---|
| 0 µg/ml | 806 |
| 10 µg/ml | 905 |
| 100 µg/ml | 1860 |

**Figure 3****: Effect of pre-incubation of high antiphospholipid antibodies (aPLs) titer serum with LysoPC and PAF on Annexin V binding to HUVECs: flows cytometry analysis after 24 hrs culture**

| Lyso PC | Mean % of APS | Median % of APS |
|---|---|---|
| 10 µg/ml | 123.6 | 121.88 |
| | 156.9 | 138.23 |
| | 169.5 | 178.5 |
| Av | 150 | 146 |
| SD | 24 | 29 |
| 100µg/ml | 114.7 | 115.4 |
| | 195.12 | 174.65 |
| | 158.1 | 175.39 |
| Av | 156 | 155 |
| SD | 40 | 34 |
| | | |

| PAF | Mean % of APS | Median % of APS |
|---|---|---|
| 10 µg/ml | 100 | 99 |
| | 155.6 | 134 |
| | 149.8 | 164 |
| Av | 135 | 132 |
| SD | 31 | 33 |
| 100µg/ml | 151 | 145 |
| | 122 | 116 |
| | 139 | 147 |
| Av | 137 | 136 |
| SD | 15 | 17 |

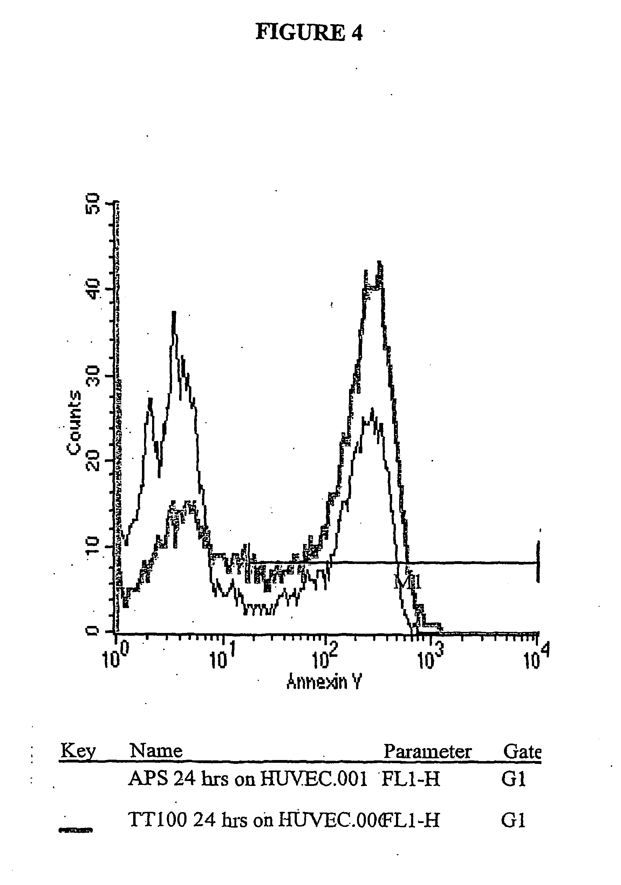

**Figure 4****: Effect of pre-incubation of high antiphospholipid antibodies (aPLs) titer serum with irrelevant antigen (tetanus toxoid) on Annexin V binding to HUVECs: flow cytometry analysis after 24 hrs culture.**

| | | | | |
|---|---|---|---|---|
| **File:** | | APS 24 hrs on HUVEC.001 | | |
| **Gated Events:** | | 10000 | | |
| **Total Events:** | | 13067 | | |

| **% Gated** | **Mean** | **Geo Mean** | **Median** | **Peak Ch** |
|---|---|---|---|---|
| 100.00 | 80.93 | 11.73 | 4.66 | 1 |
| 36.90 | 213.84 | 172.37 | 205.35 | 259 |
| | | | | |
| **File:** | | TT100 24 hrs on HUVEC.006 | | |
| **Gated Events:** | | 10000 | | |
| **Total Events:** | | 13328 | | |

| **% Gated** | **Mean** | **Geo Mean** | **Median** | **Peak Ch** |
|---|---|---|---|---|
| 100.00 | 155.14 | 47.62 | 113.42 | 1 |
| 66.69 | 230.12 | 173.93 | 212.88 | 222 |

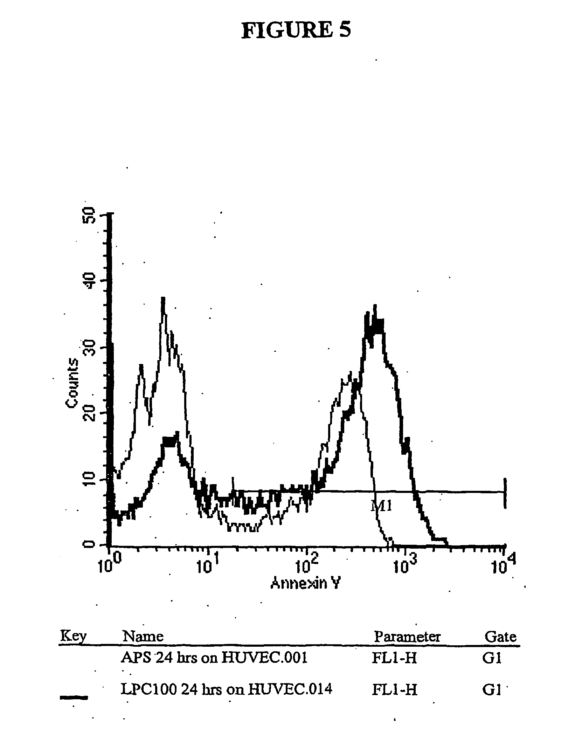

**Figure 5****: Effect of pre-incubation of high antiphospholipid antibodies (aPLs) titer serum with LysoPC on Annexin V binding to BHVECs: flow cytometry analysis after 24 hrs culture.**

| | | | | |
|---|---|---|---|---|
| **File:** | | APS 24 hrs on HUVEC.001 | | |
| **Gated Events:** | | 10000 | | |
| **Total Events:** | | 13067 | | |

| **% Gated** | **Mean** | **Geo Mean** | **Median** | **Peak Ch** |
|---|---|---|---|---|
| 100.00 | 80.93 | 11.73 | 4.66 | 1 |
| 36.90 | 213.84 | 172.37 | 205.35 | 259 |
| | | | | |
| **File:** | | LPC100 24 hrs on HUVEC.014 | | |
| **Gated Events:** | | 10000 | | |
| **Total Events:** | | 13163 | | |

| **% Gated** | **Mean** | **Geo Mean** | **Median** | **Peak Ch** |
|---|---|---|---|---|
| 100.00 | 275.37 | 62.95 | 153.99 | 1 |
| 67.03 | 408.50 | 268.96 | 352.27 | 453 |

### EXPERIMENTAL

### Study group

The study group consisted of 26 women with SLE who had survived one or more manifestations of cardiovascular disease, defined as thromboembolic, not hemorrhagic or vasculitic stroke (n=15), (confirmed by computed tomography or magnetic resonance imaging); myocardial infarction (n=7), (confirmed by electrocardiography and a rise in creatine kinase); angina pectoris (n=9) (confirmed by exercise stress test) or intermittent claudication (n=4) (peripheral atherosclerosis confirmed by angiogram),
26 age-matched women with SLE and no clinical manifestations of cardiovascular disease and 26 age-matched healthy population-based women.

All patients fulfilled the 1982 revised criteria of the American Rheumatism Association for SLE16. The study was approved by the Ethics Committee of the Karolinska Hospital. All participants gave informed consent before entering the study.

### Carotid Ultrasound

The right and left carotid arteries were examined with a duplex scanner (Acuson Sequoia, Mountain View, California, USA) and the degree of atherosclerosis was determined by intima-media thickness (IMT) was determined.

### Culture of endothelial cells binding of Annexin V

Cryopreserved pooled human umbilical venous endothelial cells (HUVECs) at passage 2 were purchased from Cascade Biologics, Inc. (Portland, OR, USA) the cultures were maintained in EGM™ phenol red-free medium (Clonetics, San Diego, CA, USA), containing 2% of fetal bovine serum and supplements. The cells were incubated in 75 cm2 flasks (TPP, AG, Trasadingen, Switzerland) under humidified 5% CO2 in 37°C conditions. All experiments were performed at passage 3 to 4. HUVECs were seeded at 2x10⁴ cells / ml density in 12-well plates (NUNC, Inc, Naperville, IL, USA) for flow cytometry analysis; at density of 1x10⁴ cells /well/100 µl in 96-well plate (TPP) for MTT assay; at 8x10³ cells/ml density in 24-well plates (NUNC) for DNA fragmentation ELISA. After allowing 12-24 hours for attachment and careful washing with serum-free medium (SFM), the cells were made quiescent in SFM for at least 12 hrs prior to treatment. Heparin-preserved plasma from the study groups was added to the monolayer at concentration of 10% in SFM.

The cells were harvested non-enzymatically with Cell Dissociation Solution (CDS; Sigma-Aldrich, St. Louis, MO, USA). HUVECs were carefully pooled with supernatants, to exclude selective loss of detached floating EC, and centrifuged at 1200 rpm for 7 min. After resuspension in 100µl of Annexin V-binding buffer (Molecular Probes Inc, Eugene, OR, USA) samples were stained with 5mg/ml of Annexin V-FITC (Mol. Probes) and incubated for 15 min on ice. Shortly before acquisition 1mg/ml of propidium iodide (PI; R&DSystems Europe Ltd, Abingdon, UK) was added. Analysis was performed on a FACScan flow cytometer (BD Biosciences, San Jose, CA, USA) equipped with CellQuest™ software. During acquisition a gate was set to exclude events smaller than 230 on linear FCS and SSC scale. For each sample 10000 events were collected.

### Immunohistochemical staining of human atherosclerotic plaque

Immunostaining was performed on human plaques, characterized previously. Plaques were collected from 12 patients undergoing carotid endarterectomy after transient ischemic attacks. All specimens contained advanced atherosclerotic lesions. As a control macroscopically healthy mesenteric artery was obtained after unrelated bowel resection. The cryostat sections were fixed for 20 minutes in 2% paraformaldehyde in PBS (Sigma Chemicals) at 4°C and stored at -70°C. After blocking endogenous peroxidase, the sections were incubated overnight with monoclonal anti-Annexin V antibody (Alexis Biochemicals, Corp., Lausen, Switzerland) of mouse type IgG2a, anti-CD68 (DakoCytomation, Glostrup, Denmark) or anti-CD31 (Monosan, Uden, TheNetherlands). Irrelevant mouse IgG2a (Serotec Ltd, Oxford, UK) served as negative control. All antibodies were diluted in 1%BSA-0.02%NaN3in PBS. After washing, 1% normal horse serum in PBS was used. Secondary antibody-biotinylated horse anti-mouse immunoglobulin (Vector Laboratories, Burlingame, CA, USA) was added. The ABC peroxidase Elite™ kit was used (Vector Laboratories). The staining was revealed with diaminobenzidine (Vector Laboratories) and counterstaining was done with haematoxyline. All sections were analyzed on a Leica DMRXA microscope (Leica, Wetzlar, Germany)

### Preparation of aPC

Total IgM or IgG fraction was separated from commercially available pooled human immunoglobulin (Gammagard®) at 50mg/ml using HiTrap IgM or IgG columns (Amersham Biosciences). Antibodies against phosphorylcholine (PC) were eluted after loading IgM or IgG fraction on NHS-Sepharose columns coupled to PC conjugated either to keyhole limpet haemocyanin protein (KLH)(1or 5 mg/ml) or to bovine serum albumin (BSA) (1 mg/ml) followed by BSA-only column. PC-BSA (Phosphoryleholine-Bovine Serum Albumin) and PC-KLH was purchased from Biosearch Technologies, INC (Ca, USA). Eluted fractions were buffer-exchanged on PD-10 columns and concentrated with Millipore Centricone® devices. Procedures were performed according to instructions given by manufacturers. The concentration of IgM aPC prepared was typically 50 µg/ml, and the concentration of IgG aPC was typically 30µg/ml.

### Annexin V binding to endothelial cells

Heparin-preserved plasma with high capacity to inhibit Annexin V binding was added to HUVECs monolayer at concentration of 10% in SFM. After 24 hrs cells were harvested with Cell Dissociation Solution (CDS; Sigma-Aldrich, St. Louis, MO, USA) and carefully pooled with supernatants, to exclude selective loss of detached floating cells, centrifugation at 1200 rpm for 7 min followed. After resuspension in 100µl of annexin V-binding buffer (Molecular Probes Inc, Eugene, OR, USA) samples were stained with 2µl of 5 mg/ml annexin V-FITC (Molecular Probes) and incubated for 15 min on ice. Shortly before acquisition 1 mg/ml of propidium iodide (PI; a vital dye; R&DSystems Europe Ltd, Abingdon, UK) was added. Analysis was performed as described above.

### RESULTS

### Effect of immunoglobulin IgG depletion on Annexin V-binding

Depletion of IgG subclass of immunoglobulins resulted in up to 2.7-2.6 fold increase in fluorescence intensity of Annexin V-binding (complete serum mean FI :267.95± vs. 709.91±; median FI: 222.67± vs 567.42±). Reconstitution of IgG fraction to the depleted sera decreased the fluorescence intensity while the cultures with IgG eluate resulted in fluorescence intensity of Annexin V binding comparable with that of complete sera.

Binding of Annexin V was significantly lower after 24 hrs when plasma from SLE cases was used as compared to controls (SLE cases vs population controls: p=0.002, SLE cases vs SLE controls p=0.02). Depletion of total IgG from sera with a high capacity to inhibit binding of Annexin V restored this binding completely. There was a striking positive association between Annexin V-binding and degree of atherosclerosis (R=0.73, p<0.001) among SLE cases. Immunostaining revealed presence of Annexin V in 11/12 plaques tested.

### Protein G affinity column chromatography

Pooled sera with a high ability to inhibit Annexin V binding to EC were 0.45 µm filtered and diluted with equal volumes of endothelial basal medium. The HiTrap Protein G HP, 1ml column with binding capacity 25 mg human IgG / ml gel from Amersham Biosciences (Uppsala, Sweden) was used according to manufacturer's instructions. The IgG fraction was obtained by eluting the column with 0.1M glycine-HCl, pH 2.7. For neutralization 1 M Tris-HCl, pH 9.0 was used. Complete serum, effluate and eluate were used for incubation with HUVEC on the day of separation at 1:10 dilution in SFM.

### Measurements of Annexin V binding to endothelial cells

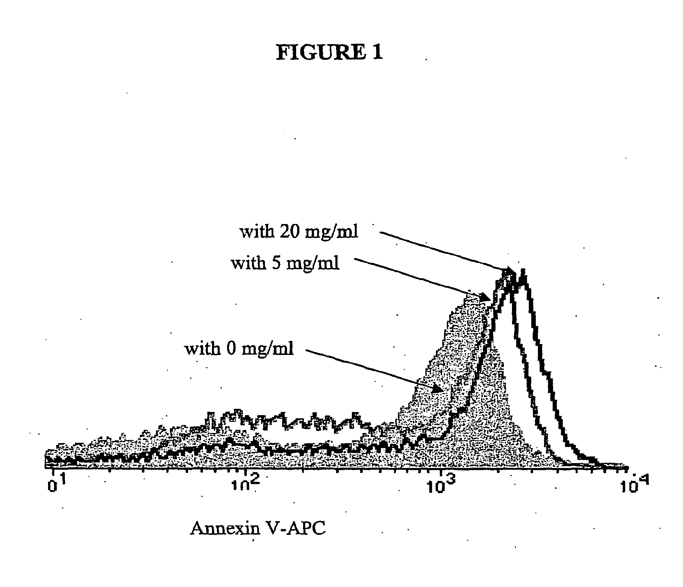

The frequency of HUVECs positive for Annexin V staining was determined either as percentage of annexin V⁺/PI⁻ cells on a bivariate dot plot or percentage of Annexin V⁺ cells based on a histogram. Annexin V-binding to HUVECs in the presence of serum known to decrease binding and preincubated with IVIG was determined. Preincubation with IVIG could restore binding of Annexin, indicating that antibodies present in IVIG could neutralise binding (Figure 1).

aPC-BSA and aPC-KLH were both associated significantly in SLE patients with a history of CVD with Annexin V binding to EC (r=0.45; p=0.02, and =0.03 respectively). aPC-BSA and aPC-KLH levels were assessed using standard techniques, for example using the following reagents. Polysorp F96 microtiter immuno-plates were purchased from Nunc ( Roskilde Denmark ), PC-BSA (Phosphorylcholine-Bovine Serum Albumin) was purchased from Biosearch Technologies, INC (USA). Bovine serum albumin (BSA), Alkaline phosphatase conjugated goat anti-human IgG(r-chain specific) , Alkaline phosphatase substrate), were obtained from Sigma (St. Louis, MO, USA). For example, IgG and IgM antibodies to PC-BSA were determined by enzyme-linked immunosorbent assay (ELISA). Pooled serum from 17 antiphospholipid syndrome patients was used as an internal standard and tested on every plate. The plateau of antibody binding was reached with the antigen concentration of 10 µg/ml. F96 microliter polysorp plate was therefore coated with PC-BSA (10µg/ml) 50 µl/well in PBS. Coated plates were incubated overnight at 4°C. After five washings with PBS, the plates were blocked with 2% BSA-PBS for 2h at room temperature and washed as described above. Serum samples were diluted (1:30) in 0.2% BSA-PBS and added at 50µl/well.

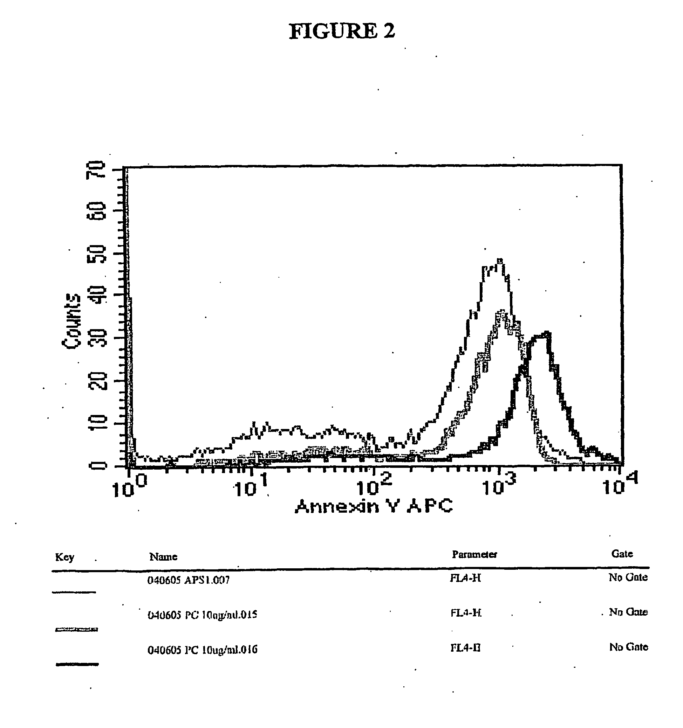

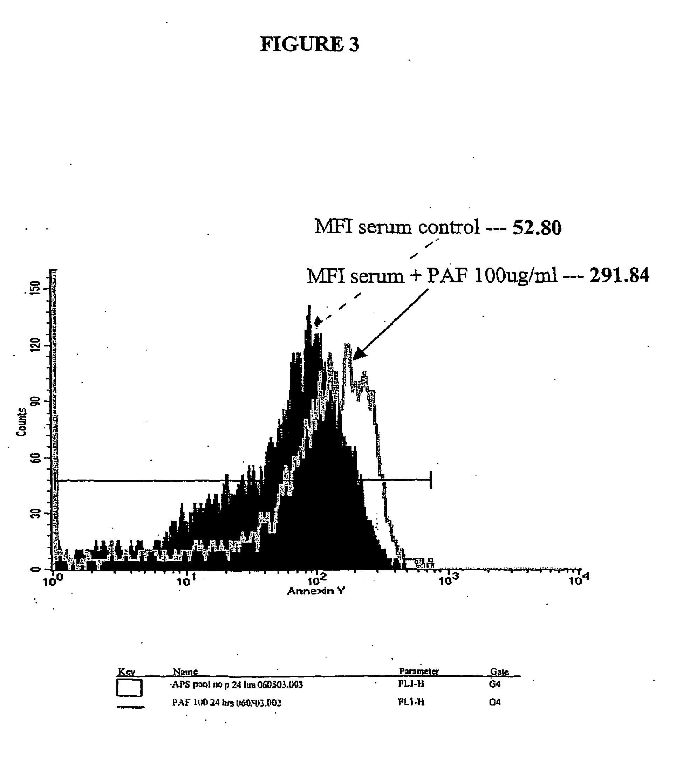

Preincubation with pneumococcal vaccine (Statens Serum Institute, Denmark), PAF, phosphatidylserine or lysophosphatidylcholine of serum with high capacity to induce decreased binding had the effect of causing decreased serum binding to antigen; and also restored Annexin V binding (Figures 2, 3, 5). In contrast, PC did had no significant effect. This suggests that antibodies binding to pneumococcal vaccine, PAF, phosphatidylserine, lysophosphatidylcholine may be involved in reducing Annexin V binding to endothelial cells.

In conclusion, Annexin V is present in atherosclerotic lesions at many sites, especially those that are prone to plaque rupture. When Annexin V binding is not optimal but instead decreased as a consequence of antibodies interfering with Annexin-plaque binding, the risk of atherothrombosis and plaque rupture is strongly raised. The restoring of the Annexin V- binding is therefore a possible novel therapy for atherothrombosis and especially plaque rupture, the main cause of cardiovascular disease. Two methods are provided by this invention. One is based on the use of an optimal dose of Annexin V or a salt of the protein, which preferably should be administered by intravenous injections, the other is based on the use of ready available immunoglobulins, or an affinity purified subfraction of the immunoglobulins, which can also be administered by injections.

The effective amount of Annexin V (or immunoglobulins) in the dose can be determined from a diagnostic status analysis on the current Annexin V- plaque binding. The binding was determined using the analysis method given above. The treatment using a pharmaceutical composition comprising the active component is preferably administered to subjects at risk A subject at risk is an SLE patient with frequent repeating atherothrombosis. Another subject at risk is a patient who has, or has had, or is at risk of, a pneumococcal infection.

### REFERENCES

1. Ross R. Atherosclerosis-an inflammatory disease. N Engl J Med. 1999;340:115-26.
2. Frostegard J, Ulfgren AK, Nyberg P, Hedin U, Swedenborg J, Andersson U, Hansson GK. Gytokine expression in advanced human atherosclerotic plaques: dominance of pro-inflammatory (Th1) and macrophage-stimulating cytokines. Atherosclerosis. 1999;145:33-43.
3. Manzi S, Selzer F, Sutton-Tyrrell K, Fitzgerald SG, Rairie JE, Tracy RP, Kuller LH. Prevalence and risk factors of carotid plaque in women with systemic lupus erythematosus. Arthritis Rheum. 1999;42:51-60.
4. Petri M, Roubenoff R, Dallal GE, Nadeau MR, Selhub J, Rosenberg IH. Plasma homocysteine as a risk factor for atherothrombotic events in systemic lupus erythematosus. Lancet. 1996;348:1120-4.
5. Svenungsson E, Jensen-Urstad K, Heimburger M, Silveira A, Hamsten A, de Faire U, Witztum JL, Frostegard J. Risk factors for cardiovascular disease in systemic lupus erythematosus. Circulation. 2001;104:1887-93.
6. Svenungsson E, Fei GZ, Jensen-Urstad K, de Faire U, Hamsten A, Frostegard J. TNF-alpha: a link between hypertriglyceridaemia and inflammation in SLE patients with cardiovascular disease. Lupus. 2003;12:454-61.
7. Svenungsson E GI, Fei G, Lundberg IE, Klareskog L, Frostegard J. Blood lipids and TNF-a are closely related markers of Disease Activity and Disease Damage in Systemic Lupus Erythematosus. Arthritis&Rheumatism in press. 2003.
8. Hughes G. Thrombosis, abortion, cerebral disease and the lupus antikoagulant. British Medical Journal. 1983;287:1088-1089.
9. Asherson RA, Khamashta MA, Ordi-Ros J, Derksen RH, Machin SJ, Barquinero J, Outt HH, Harris EN, Vilardell-Torres M, Hughes GR. The "primary" antiphospholipid syndrome: major clinical and serological features. Medicine (Baltimore). 1989;68:366-74.
10. Wu R, Lemne C, De Faire U, Frostegard J. Antibodies to platelet-activating factor are associated with borderline hypertension, early atherosclerosis and the metabolic syndrome. J Intern Med. 1999;246:389-97.
11. Vaarala O, Mänttäri M, Manninen V, Tenkanen L, Puurunen M, Aho K, T P. Anti-Cardiolipin Antibodies and Risk of Myocardial Infarction in a Prospective Cohort of Middle-Aged Men. Circulation. 1995;91:23-27.
12. Rand JH, Wu XX, Quinn AS, Chen PP, McCrae KR, Bovill EG, Taatjes DJ. Human monoclonal antiphospholipid antibodies disrupt the Annexin A5 anticoagulant crystal shield on phospholipid bilayers: evidence from atomic force microscopy and functional assay. Am J Pathol. 2003;163:1193-200.
13. Rand JH, Wu XX, Guller S, Scher J, Andree HA, Lockwood CJ. Antiphospholipid immunoglobulin G antibodies reduce Annexin-V levels on syncytiotrophoblast apical membranes and in culture media of placental villi. Am J Obstet Gynecol. 1997;177:918-23.
14. Rand JH. Antiphospholipid antibody-mediated disruption of the Annexin-V antithrombotic shield: a thrombogenic mechanism for the antiphospholipid syndrome. J Autoimmun. 2000;15:107-11.
15. Stratton et al.,Circulation 92: 3113-3121 (1995); Thiagarajan and Benedict, Circulation 96: 23392347 (1997)
16. Reno and Kasina, International Patent Application PCT/US95/07599 (WO 95/34315).
17. Romisch et al., Thrombosis Res. 61: 93-104 (1991).
18. Blankenberg FG, Narula J, Strauss HW, Ghazarossian V. US Patent Application US 2003/152513 A1.
19. Mari C, Blankenberg F, Narula Z, Narula J, Ghazarossian V, Tait J, Strauss HW. Annexin V, a New Therapeutic Tool in Atherosclerosis. Proceedings of the SNM 49th Annual Meeting. 2002;43:7P.
20. Thiagarajan P, Benedict CR. Inhibition of Arterial Thrombosis by Recombinant Annexin V in a Rabbit Carotid Artery Injury Model. Circulation. 1997;96:2339-2347.

## Claims

1. Use of the Annexin V protein, optionally in the form of a salt, in the manufacture of a pharmaceutical composition to prevent plaque rupture in humans.

2. A pharmaceutical composition comprising the Annexin V protein, optionally in the form of a salt, for use in the prevention of plaque rupture in humans.

3. The use according to claim 1 or the pharmaceutical composition according to claim 2, wherein the pharmaceutical composition comprises an effective amount of the Annexin V protein, optionally in combination with a carrier and additives.

4. The use according to claim 1 or claim 3 or the pharmaceutical composition according to claim 2 or claim 3, wherein the effective amount of the Annexin V in the pharmaceutical composition is determined from a diagnostic status analysis of the Annexin V-endothelium binding.

5. A use according to any one of claims 1, 3 or 4 or the pharmaceutical composition according to any one of claims 2 to 4 to prevent plaque rupture in a systemic lupus erythematosus (SLE) patient.

6. Use of an intravenous immunoglobulin (IVIG) preparation in the manufacturing of a medicament to prevent plaque rupture in a human SLE patient.

7. An intravenous immunoglobulin (IVIG) preparation for use in preventing plaque rupture in a human SLE patient.

8. Use of a purified subfraction of a pooled immunoglobulin preparation with the capacity to decrease the inhibitory effect of plasma of a systemic lupus erythematosus (SLE) patient on binding of Annexin V to endothelial cells, in the manufacturing of a medicament to prevent plaque rupture in a human SLE patient, wherein the purified subfraction is -
(a) a purified anti Ig antibody subfraction containing anti-aPAF antibodies, and/or
(b) a purified anti Ig antibody subfraction containing anti-IgG antibodies.

9. A purified subfraction of a pooled immunoglobulin preparation with the capacity to decrease the inhibitory effect of plasma of a systemic lupus erythematosus (SLE) patient on binding of Annexin V to endothelial cells, for use in preventing plaque rupture in a human SLE patient, wherein the purified subfraction is -
(c) a purified anti Ig antibody subfraction containing anti-aPAF antibodies, and/or
(d) a purified anti Ig antibody subfraction containing anti-IgG antibodies.

10. A use according to any one of claims 1, 3 to 6 or 8, the pharmaceutical composition according to any one of claims 2 to 5, the IVIG preparation according to claim 7 or the purified subfraction of a pooled immunoglobulin preparation according to claim 9 for preventing plaque rupture in a patient with vulnerable plaques.

## Patentansprüche

1. Verwendung des Annexin V-Proteins, optional in der Form eines Salzes, bei der Herstellung einer pharmazeutischen Zusammensetzung, um Plaqueruptur bei Menschen zu verhindern.

2. Pharmazeutische Zusammensetzung, die das Annexin V-Protein aufweist, optional in der Form eines Salzes, zur Verwendung bei der Prävention von Plaqueruptur bei Menschen.

3. Verwendung nach Anspruch 1 oder pharmazeutische Zusammensetzung nach Anspruch 2, wobei die pharmazeutische Zusammensetzung eine wirksame Menge des Annexin V-Proteins aufweist, optional in Kombination mit einem Träger und Zusätzen.

4. Verwendung nach Anspruch 1 oder Anspruch 3 oder pharmazeutische Zusammensetzung nach Anspruch 2 oder Anspruch 3, wobei die wirksame Menge des Annexin V in der pharmazeutischen Zusammensetzung aus einer diagnostischen Statusanalyse der Annexin V-Endothelium-Bindung bestimmt wird.

5. Verwendung nach einem der Ansprüche 1, 3 oder 4, pharmazeutische Zusammensetzung nach einem der Ansprüche 2 bis 4, um Plaqueruptur bei einem Patienten mit systemischem Lupus erythematosus (SLE) zu verhindern.

6. Verwendung einer intravenösen Immunglobulin-(IVIG-)Präparation bei der Herstellung eines Medikaments, um Plaqueruptur bei einem menschlichen SLE-Patienten zu verhindern.

7. Intravenöse Immunglobulin-(IVIG-)Präparation zur Verwendung beim Verhindern von Plaqueruptur bei einem menschlichen SLE-Patienten.

8. Verwendung einer gereinigten Unterfraktion einer gepoolten Immunglobulinpräparation mit der Kapazität, den inhibitorischen Effekt von Plasma eines Patienten mit systemischem Lupus erythematosus (SLE) auf das Binden von Annexin V an Endothelialzellen herabzusetzen, bei der Herstellung eines Medikaments, um Plaqueruptur bei einem menschlichen SLE-Patienten zu verhindern, wobei die gereinigte Unterfraktion ist
(a) eine gereinigte Anti-Ig-Antikörper-Unterfraktion, die Anti-aPaF-Antikörper enthält, und/oder
(b) eine gereinigte Anti-Ig-Antikörper-Unterfraktion, die Anti-IgG-Antikörper enthält.

9. Gereinigte Unterfraktion einer gepoolten Immunglobulinpräparation mit der Kapazität, den inhibitorischen Effekt von Plasma eines Patienten mit systemischem Lupus erythematosus (SLE) auf das Binden von Annexin V an Endothelialzellen herabzusetzen, zur Verwendung beim Verhindern von Plaqueruptur bei einem menschlichen SLE-Patienten, wobei die gereinigte Unterfraktion ist
(c) eine gereinigte Anti-Ig-Antikörper-Unterfraktion, die Anti-aPaF-Antikörper enthält, und/oder
(d) eine gereinigte Anti-Ig-Antikörper-Unterfraktion, die Anti-IgG-Antikörper enthält.

10. Verwendung nach einem der Ansprüche 1, 3 bis 6 oder 8, pharmazeutische Zusammensetzung nach einem der Ansprüche 2 bis 5, IVIG-Präparation nach Anspruch 7 oder gereinigte Unterfraktion einer gepoolten Immunglobulinpräparation nach Anspruch 9 zum Verhindern von Plaqueruptur bei einem Patienten mit verletzlichen Plaques.

## Revendications

1. Utilisation de la protéine Annexine V, éventuellement sous la forme d'un sel, dans la fabrication d'une composition pharmaceutique pour prévenir une rupture de plaque chez l'être humain.

2. Composition pharmaceutique comprenant la protéine Annexine V, éventuellement sous la forme d'un sel, pour une utilisation dans la prévention d'une rupture de plaque chez l'être humain.

3. Utilisation selon la revendication 1 ou composition pharmaceutique selon la revendication 2, laquelle composition pharmaceutique comprenant une quantité efficace de la protéine Annexine V, éventuellement en combinaison avec un véhicule et des additifs.

4. Utilisation selon la revendication 1 ou la revendication 3 ou composition pharmaceutique selon la revendication 2 ou la revendication 3, où la quantité efficace de l'Annexine V dans la composition pharmaceutique est déterminée par une analyse de l'état diagnostique de la liaison Annexine V-endothélium.

5. Utilisation selon l'une quelconque des revendications 1, 3 ou 4, ou composition pharmaceutique selon l'une quelconque des revendications 2 à 4, pour prévenir une rupture de plaque chez un patient atteint de lupus érythémateux systémique (SLE).

6. Utilisation d'une préparation intraveineuse d'immunoglobulines (IVIG) dans la fabrication d'un médicament pour prévenir une rupture de plaque chez un patient humain atteint de SLE.

7. Préparation intraveineuse d'immunoglubulines (IVIG) pour une utilisation dans la prévention d'une rupture de plaque chez un patient humain atteint de SLE.

8. Utilisation d'une sous-fraction purifiée d'une préparation d'immunoglobulines regroupées ayant la capacité à diminuer l'effet inhibiteur du plasma d'un patient atteint de lupus érythémateux systémique (SLE) sur la liaison de l'Annexine V aux cellules endothéliales, dans la fabrication d'un médicament pour prévenir une rupture de plaque chez un patient humain atteint de SLE, dans laquelle la sous-fraction purifiée est :
(a) une sous-fraction d'anticorps anti-Ig purifiée contenant des anticorps anti-aPAF, et/ou
(b) une sous-fraction d'anticorps anti-Ig purifiée contenant des anticorps anti-IgG.

9. Sous-fraction purifiée d'une préparation d'immunoglobulines regroupées ayant la capacité à diminuer l'effet inhibiteur du plasma d'un patient atteint de lupus érythémateux systémique (SLE) sur la liaison de l'Annexine V aux cellules endothéliales, pour une utilisation dans la prévention d'une rupture de plaque chez un patient humain atteint de SLE, laquelle sous-fraction purifiée étant :
(c) une sous-fraction d'anticorps anti-Ig purifiée contenant des anticorps anti-aPAF, et/ou
(d) une sous-fraction d'anticorps anti-Ig purifiée contenant des anticorps anti-IgG.

10. Utilisation selon l'une quelconque des revendications 1, 3 à 6 ou 8, composition pharmaceutique selon l'une quelconque des revendications 2 à 5, préparation IVIG selon la revendication 7 ou sous-fraction purifiée d'une préparation d'immunoglobulines regroupées selon la revendication 9, pour prévenir une rupture de plaque chez un patient ayant des plaques vulnérables.
